# EUROPÄISCHE PATENTSCHRIFT

(11) **EP 1 198 713 B1**
(45) Veröffentlichungstag und Bekanntmachung des Hinweises auf die Patenterteilung: **25.01.2006**
(21) Anmeldenummer: 00954386.9
(22) Anmeldetag: 28.07.2000
(51) Int. Cl.: G01N 33/569, C12Q 1/04

(54) **NACHWEIS UND IDENTIFIKATION VON BAKTERIENSTÄMMEN**
DETECTION AND IDENTIFICATION OF BACTERIAL STRAINS
MISE EN EVIDENCE ET IDENTIFICATION DE SOUCHES BACTERIENNES

(30) Priorität: 30.07.1999 DE 19936047
(43) Veröffentlichungstag der Anmeldung: 24.04.2002
(73) Patentinhaber: Profos AG, 93053 Regensburg (DE)
(72) Erfinder: MILLER, Stefan, D-93059 Regensburg (DE)
(74) Vertreter: Dehmel, Albrecht
(86) Internationale Anmeldenummer: PCT/DE2000/002495
(87) Internationale Veröffentlichungsnummer: WO 2001/009370

(56) Entgegenhaltungen:
- WO-A-93/17129
- WO-A-94/06931
- WO-A-97/22713
- WO-A-98/48042
- US-A- 5 824 468

## Beschreibung

Die vorliegende Erfindung betrifft ein Verfahren zum Nachweis von Bakterien, umfassend die folgenden Schritte: Kopplung von Bakteriophagen und/oder Bakterienphagenproteinen an einen Träger, Inkubieren des mit den Bakteriophagen und/oder Bakterienphagenproteinen gekoppelten Trägers mit einer Probe, gegebenenfalls Entfernen der Probe und der nicht an die Bakteriophagen und/oder Bakterienphagenproteine gebundenen Bakterien der Probe, gegebenenfalls Zugabe von die Bakterienmembran permeabilisierenden oder zerstörenden Substanzen, und Nachweisen der an die Bakteriophagen und/oder Bakterienphagenproteine gebundenen Bakterien der Probe, wobei die gebundenen Bakterien keinem Kultivierungsschritt unterzogen werden.

Der schnelle und exakte Nachweis von Bakterien ist der erste, notwendige Schritt zur Diagnose und Behandlung einer bakteriellen Infektion bei Mensch und Tier sowie zur Ergreifung von Präventionsmaßnahmen. Ferner dient der Nachweis zur Kontrolle der Hygiene und Qualität von Rohprodukten und verarbeiteten Nahrungmitteln sowie zur Kontrolle der Hygiene und Qualität von Trink- und Brauchwasser und der Wasserqualität von öffentlichen Bädern. Darüber hinaus dient der Nachweis dem Prozeßmonitoring und -Optimierung, sowie der Qualitätskontrolle in der Umweltanalytik. Im Gegensatz zu den meisten bisher verwendeten Verfahren, ermöglicht die hier beschriebene Methode auch einen einfachen Nachweis vor Ort.

Der Nachweis von Bakterien erfolgt aus biologischen Proben zumeist mit Hilfe einer Kombination von Kulturmethoden unter Verfolgung von Stoffwechselaktivitäten. Bei der Phagen-Typisierung (engl.: "phage-typing") von Bakterienstämmen einer Bakterienart werden Kulturmethoden mit der Empfindlichkeit von Bakterien gegen Typisierungs-Bakteriophagen gekoppelt. Dabei wird ein dichter Bakterienrasen auf einer Agarplatte der zu untersuchenden Probe, der durch Isolierung einer Einzelkolonie und anschliessender Vermehrung derselben gewonnen wurde, mit Suspension aus Bakteriophagen in Softagar überschichtet. Das Ergebnis wird nach Übemachtinkubation bei der optimalen Wachstumstemperatur der Bakterien, die üblicherweise zumeist 37°C beträgt, durch Auszählen der Plaques und der Kontrolle der Plaquemorphologie erhalten. Eine Variante der Typisierung sieht die Messung von Adenylatkinase nach Phagen-vermittelter Zelllyse vor. Dabei wird eine Übernachtkultur der zu untersuchenden Bakterien in Puffer verdünnt, mit Phagen versetzt und die Lyse durch spezifische Phagen per Adenylatkinaseaktivität gemessen.

In allen bisher beschriebenen Verfahren wird die Lyse abgewartet, bevor der Nachweis erfolgt, bzw. der Nachweis wird per Lyse geführt. Dadurch können Infektquellen verfolgt und Infektionsquellen aufgefunden werden. Diese Typisierung ist bei einer Reihe von Bakterien wie *Salmonella typhi, Salmonella paratyphi B, Staphylocuccus aureus, Pseudomonas aeruginosa, sowie einer Vielzahl weiterer Bakterien* seit Jahren etabliert. Diese etablierten Nachweisverfahren liefern Ergebnisse jedoch meist erst nach mehreren Tagen. Allerdings ist gerade die schnelle und genaue Bestimmung der Bakterienart (Typisierung) für eine rasche Reaktion sehr oft von großer Bedeutung.

WO 93/17129 beschreibt ein Verfahren zum Nachweis bestimmter Bakterien in einer Probe, wobei die Bakterien mittels eines Bakteriophagen nachgewiesen werden, der in der Lage ist, an das Bakterium zu binden und an den ein Bindungspartner eines zweiteiligen Bindungspaares befestigt ist. Der zweite Bindungspartner des zweiteiligen Bindungspaares ist an einen wasserunlöslichen, in Wasser suspendierbaren festen Träger gebunden. Die Bakterien werden bei Anwesenheit in der Probe von den Bakteriophagen gebunden, die dann über die Bindungspartner an den festen Träger gebunden werden.

Neuerdings werden auch schnellere molekularbiologische Nachweisverfahren z.B. die Polymerasekettenreaktion angewendet, die jedoch den Nachteil haben, kontaminationsanfälliger zu sein. Auch bei diesen Verfahren steht das Ergebnis üblicherweise erst nach einem Tag zur Verfügung.

Weiterhin ist für eine Identifizierung der Bakteriengattung in manchen Fällen die Einsendung von Proben an hoch spezialisierte Referenzlabors notwendig, was ebenfalls einen zeitaufwendigen und kostenintensiven Faktor darstellt.

Daher liegt der Erfindung die Aufgabe zu Grunde, ein schnelles und kostengünstiges Nachweisverfahren für Bakterien bereitzustellen, das einerseits von speziell mikrobiologisch geschulten Personen im Labor, andererseits auch in einer vereinfachten Form an Ort und Stelle und ohne entsprechende Vorkenntnisse durchgeführt werden kann.

Die Aufgabe wird durch den in den Patentansprüchen definierten Gegenstand gelöst.

Ein Aspekt der vorliegenden Erfindung ist daher ein schnelles und exaktes Nachweissystem für Bakterien, welches Informationen über die Bakterienart, über den Bakterienstamm liefert und gegebenenfalls eine Quantifizierung der Bakterien erlaubt und auf der Erkennung dieser Bakterien durch Bakteriophagen oder Bakteriophagenproteinen beruht.

Die üblichen Bakteriennachweisverfahren auf der Basis von Bakteriophagen beinhalten zeitaufwendige Kultivierungsschritte und die Bakteriophagen-vermittelten Lyse der Zellen. Das erfindungsgemäße Verfahren nutzt zwar ebenso die spezifische Zellerkennung durch Bakteriophagen aus. Im Gegensatz zu den bislang beschriebenen Verfahren schließt sich an den spezifischen Zellerkennungsschritt, nach der Abtrennung unspezifisch gebundener Bakterien, direkt ein entsprechender Bindungstest an, z.B. die Messung einer spektroskopischen (z.B. per Absorption, Fluoreszenz, Bio- oder Chemieluminiszenz, oder Circulardichroismus) oder elektrischen (z.B. per Kapazitätsmessung oder Änderung der elektrischen Leitfähigkeit) Signaländerung an. Dadurch wird ein Nachweis der Bakterien bereits nach wenigen Minuten, statt wie bisher erst nach Stunden bzw. Tagen ermöglicht. Durch eine gezielte Kopplung, insbesondere eine kovalente Fixierung von Bakteriophagen an geeignete Trägerstrukturen, z.B. Mikrotiterplatten, Teststreifen, Objektträger, Wafer, Filtermaterialien oder Durchflußzellkammern wird der Verfahrensschritt des Bindungstests durch Reduktion des unspezifischen Hintergrundes begünstigt und eine breite Anwendung für alle Bakterien ermöglicht. Die Trägerstrukturen können z.B. aus Polystyrol, Polypropylen, Polycarbonat, PMMA, Celluloseacetat, Nitrozellulose, Glas, Siliziumwafer bestehen. Der Einsatz des erfindungsgemäßen Verfahrens ermöglicht darüber hinaus auch den Einsatz lysogener Bakteriophagen zur Bakteriendetektion.

Ein Aspekt der vorliegenden Erfindung besteht daher in der Bereitstellung eines Verfahrens zum Nachweis von Bakterien, umfassend die folgenden Schritte: Kopplung von Bakteriophagen und/oder Bakterienphagenproteinen an einen Träger, Inkubieren des mit den Bakteriophagen und/oder Bakterienphagenproteinen gekoppelten Trägers mit einer Probe, gegebenenfalls Entfernen der Probe und der nicht an die Bakteriophagen und/oder Bakterienphagenproteine gebundenen Bakterien der Probe, gegebenenfalls Zugabe von die Bakterienmembran permeabilisierenden oder zerstörenden Substanzen, und Nachweisen der an die Bakteriophagen und/oder Bakterienphagenproteine gebundenen Bakterien der Probe, wobei die gebundenen Bakterien keinem Kultivierungsschritt unterzogen werden.

Bevorzugt ist ein Verfahren, wobei der Nachweis mittels eines colorimetrischen Nachweises von Zellbestandteilen und/oder Produkten der Phagenreproduktion, mittels eines Nachweises von DNA und/oder RNA oder mittels eines Immunnachweises durchgeführt wird. Ferner bevorzugt ist ein Verfahren, wobei die Bakteriophagen und/oder Bakterienphagenproteine mittels Adsorption an den Trägern oder mittels chemischer Bindung an den Träger gekoppelt sind. Ferner bevorzugt ist ein Verfahren, wobei die Bakteriophagen und/oder Bakterienphagenproteine Modifikationen aufweisen. Ferner bevorzugt ist ein Verfahren, wobei mindestens zwei verschiedene Bakteriophagen und/oder Bakterienphagenproteine verwendet werden, die mindestens zwei verschiedene Bakterienarten und/oder -gattungen erkennen. Ferner bevorzugt ist ein Verfahren, wobei der Träger z.B. eine Mikrotiterplatte, Teststreifen, Objektträger, Wafer, Filtermaterial oder eine Durchflußzellkammer ist und z.B. aus Polystyrol, Polypropylen, Polycarbonat, PMMA, Celluloseacetat, Nitrozellulose, Glas oder Siliziumwafer besteht.

Für den Nachweis werden Bakteriophagen verwendet, die für die gewünschten nachzuweisenden Bakterien spezifisch sind. Die Phagen müssen dabei nicht nur für eine Bakterienart spezifisch sein, sondern sie können auch für mehrere Bakterienarten oder für eine Bakteriengattung spezifisch sein. Welche Phagen für den Nachweis verwendet werden hängt davon ab, welche Bakterien nachgewiesen werden sollen. Ferner können auch zwei oder mehrere Phagen in einem Nachweisverfahren eingesetzt werden, um gleichzeitig mehrere Bakterienarten nachzuweisen, oder eine Bakteriengattung genau zu typisieren. Die verwendeten Bakteriophagen können kommerziell erhältliche, aus Stammsammlungen wie z.B. DSM oder ATCC, oder eigens für diesen Zweck isolierte Bakteriophagen sein. Es können sowohl lytische als auch lysogene Bakteriophagen verwendet werden, wobei lytische Phagen bevorzugt sind. Die morphologischen Eigenschaften schränken die Phagenauswahl nicht ein, jedoch sind Myoviridae (T4-ähnliche Phagen), Siphoviridae (λ-ähnliche Phagen) oder Podoviridae (T7-, P22-ähnliche Phagen) bevorzugt.

Die Phagen binden die entsprechenden Rezeptoren der Bakterien. Dabei kommt es zu einer Protein-Protein- oder Protein-Kohlenhydrat-, oder Protein-Lipid-Interaktion. Nach der hoch spezifischen Erkennung seines Wirtes injiziert der Phage seine genetische Information (Einstrang- oder Doppelstrang-DNA oder -RNA) in die Zelle und liegt entweder lysogen vor oder produziert in Falle der Lyse neue Phagenpartikel. Mit der Injektion der Phagen-Nukleinsäure wird die Bindung der Bakterien an die Phagen fixiert, in den meisten Fällen irreversibel. Nach Abschluß des Erkennungsschrittes erfolgt in dem erfindungsgemäßen Verfahren der Nachweis der Bakterien. Diese Methode ist prinzipiell für alle Bakterien anwendbar, für die Phagen beschrieben sind oder isoliert werden können. Bevorzugte Bakterien sind solche, die für die Lebensmittelindustrie, in der Medizin oder Umweltanalytik relevant sind, wie z.B. Milchsäurebakterien, z.B. Leuconostoc, Pseudomonas, und Enterobakterien, z.B. E.coli, Salmonella. Der Erkennungsschritt kann bei jeder Temperatur im Bereich von 0° bis 90°C durchgeführt werden, vorzugsweise bei einer Temperatur im Bereich von 4° bis 45°C, besonders bevorzugt bei einer Temperatur im Bereich von 15 bis 37°C, insbesonders bevorzugt bei einer Temperatur im Bereich von 20 bis 37°C, insbesondere bei RT.

Weiterhin ist es möglich für den Nachweis statt kompletter Phagen, einzelne Phagenproteine, z.B. Phagenrezeptoren, -adhäsine oder Teile dieser Adhäsine, z.B. p12 aus T4 oder p9-tailspike aus P22, oder Varianten dieser Proteine, zu isolieren und einzusetzen. Bevorzugt werden dabei Adhäsine, die irreversibel an Bakterien binden, oder deren Bakterienbindungstasche entsprechend gentechnisch oder chemisch modifiziert wurde, um eine irreversible Bindung zu erreichen. Ein Beispiel für gentechnisch veränderte Phagenproteine sind die "active-site Mutanten" des P22-Tailspike (vgl. Baxa et al., Biophys. J. Vol. 71, 2040-2048; 1996). Die Phagenproteine können wie die Bakteriophagen für das erfindungsgemäße Verfahren verwendet werden.

Die für das erfindungsgemäße Verfahren verwendeten Bakteriophagen und/oder Bakteriophagenproteine können durch eine gezielte oder zufällige (random) Mutagenese in ihrer Wirtsspezifität bzw. ihrer Bindungseigenschaften an die Trägerstrukturen angepaßt werden. Durch die Mutagenese werden Mutationen eingeführt, die Aminosäureadditionen, -deletionen,-substitutionen oder chemische Modifikationen sein können. Diese Mutationen bewirken eine Veränderung der Aminosäuresequenz in der Bindungsregion der Phagen oder Phagenproteine, mit dem Ziel, Spezifität und Bindungsaffinität an Testbedürfnisse anzupassen, z.B. die Bindung der Bakterien an die isolierten Phagenproteine irreversibel zu machen, um die Waschmöglichkeiten zu verbessern. Darüber hinaus kann eine gentechnische oder biochemische Modifikation der Phagenproteine durchgeführt werden, mit dem Ziel, die gegebenenfalls vorhandene enzymatische Aktivität auszuschalten, um dadurch die Bindung zu verbessern oder irreversibel zu machen.

Für den erfindungsgemäßen Nachweis werden die Phagen oder Phagenproteine auf geeigneten Trägerstrukturen, z.B. Mikrotiterplatten, Teststreifen, Objektträgem, Wafern, Filtermaterialien oder Durchflußzellkammern, immobilisiert. Die Trägerstrukturen können z.B. aus Polystyrol, Polypropylen, Polycarbonat, PMMA, Celluloseacetat, Nitrozellulose, Glas, Siliziumwafer bestehen. Die Immobilisierung kann durch Adsorption oder durch kovalente Bindung erreicht werden, wobei die kovalente Bindung bevorzugt wird. Wichtig hierbei ist eine funktionelle Immobilisierung, d.h. die Phagen bzw. Phagenproteine verfügen trotz Bindung an das Trägermaterial über für Bakterien zugängliche Strukturen.

Zur Unterdrückung einer unspezifischen Reaktion der zu untersuchenden Bakterien mit dem Trägermaterial kann eine Blockierung mit Rinderserumalbumin oder Tween 20 oder ähnlichen im ELISA-Bereich verwendeten Substanzen z.B. Milchpulver durchgeführt werden. Ferner kann zur Erhöhung der Effizienz der Adsorption die Trägersysteme mit geeigneten Proteinen (z.B. spezifische Antikörper gegen Phagenproteine oder unspezifische Proteine, wie z. B. Rinderserumalbumin), Peptiden, Sacchariden (z.B. Mono-, Oligo- oder Polysaccharide) oder Detergentien (z.B. Tween 20 oder Octylglukosid) vorbeschichtet werden. Diese Beschichtungen können entweder über Nacht bei einer Temperatur im Bereich von 4-20°C oder in 2-4 h bei 30-65°C durchgeführt werden. Anschließend wird die überschüssige Flüssigkeit abgenommen und die Trägerstruktur bei etwa 60-70°C getrocknet. Die Basisbeschichtung soll zum einen eine Adsorption von funktionsfähigen Phagen oder Phagenproteinen gewährleisten und zum anderen eine unspezifische Adsorption der Testbakterien an die Trägerstuktur verhindern, um die Testeffizienz zu erhöhen.

Im Anschluß an die Basisbeschichtung werden die Phagen oder Phagenproteine aufgebracht. Dazu wird eine wäßrige gepufferte Lösung der Phagen oder Phagenproteine auf die vorbehandelte Trägerstruktur aufgetragen. Nach einer Adsorption bei 4-20°C über Nacht oder bei 30-65°C für 2-4 h wird die Beschichtungslösung abgenommen und die Trägerstruktur wie oben beschrieben getrocknet. Zu einer Erhöhung der Beschichtungseffizienz kann nachträglich eine kovalente Fixierung der Phagen oder Phagenproteine mit chemischen Crosslinkem, wie z.B. Glutaraldehyd, durchgeführt werden.

Zur Verbesserung der funktionellen Immobilisierung kann bei den verwendeten Phagen, z.B. bei Myoviridae, Siphoviridae und Podoviridae, ein Phage-Display-Ansatz verwendet werden (vgl. Gene, 1998, 215, 439-444), wobei Peptide auf den Phagenkopf- oder Kapsidproteinen exprimiert werden, die über definierte Bindungseigenschaften für bestimmte Trägersysteme verfügen.

Die Immobilisierung der Phagen und Phagenproteine an das Trägermaterial mittels Adsorption kann durch Inkubation einer Phagenlösung in wässrigem Puffer, z.B. 100mM Tris pH 7.3 oder 100mM Natriumphosphat pH 7.5, über mehrere Stunden oder über Nacht bei 5°C bis 45°C, vorzugsweise bei 15°C bis 37°C, besonders bevorzugt bei 20°C bis 37°C, insbesondere bevorzugt bei RT, durchgeführt werden.

Ferner müssen die Phagen oder Phagenproteine nicht direkt auf dem Träger immobilisiert werden, sondern können an Polypeptide binden, die ihrerseits auf dem Träger immobilisiert wurden. Diese Polypeptide können für die Phagen oder Phagenproteine spezifisch Antikörper, Lektine, Rezeptoren oder Anticaline sein.

Bei der Immobilisierung der Phagen und Phagenproteine mittels kovalenter Kopplung können aufgrund von stringenteren Waschbedingungen unspezifisch gebundene Bakterien besser entfernt werden. Bei der kovalenten Kopplung können die Phagen und Phagenproteine z.B. über primäre Aminogruppen oder Carboxylgruppen an bereits vom Hersteller aktivierte Trägermaterialien, z.B. Mikrotiterplatten von Nunc, Xenobind oder Costar, über Standardbedingungen, z.B. -NH₂ über Cyanurylchlorid (Russian Chemical Rev., 1964, 33: 92-103), oder -COO- über EDC (1-Ethyl-3'[3'Dimethylaminopropyl]carbodiimid) (Anal. Biochem. 1990, 185: 131-135) gekoppelt werden. Darüberhinaus können die Trägermaterialien mit geeigneten Verfahren direkt aktiviert werden. Eine Möglichkeit, die wegen der Anwendbarkeit für ein breites Spektrum an Trägermaterialien bevorzugt wird, ist die Silanisierung des Trägermaterials. Beispielsweise kann die Silanisierung bei Polystyrol durch Flammpyrolyse durchgeführt werden. Anschliessend werden geeignete Haftvermittler aufgetragen, die eine Kopplung über z.B. primäre Aminogruppen oder Carboxylgruppen ermöglichen.

Um eine gerichtete Immobilisierung, z.B. bei T4-Phagen eine Kopplung über den "Kopf" an den Träger, zu erreichen, können quellbare Polymere, mit Poren definierter Größe oder auf Metalloberflächen auch Mischungen verschieden langer Alkylthiole eingesetzt werden.

Zur Bindung der zu untersuchenden Bakterien an die immobilisierten Bakteriophagen oder Phagenproteine wird die Untersuchungsprobe in wäßriger Form mit den Phagen oder Phagenproteinen in Kontakt gebracht und inkubiert. Die Inkubation erfolgt bei einer Temperatur im Bereich von 4° bis 90°C, vorzugsweise bei einer Temperatur im Bereich von 4° bis 45°C, besonders bevorzugt bei einer Temperatur im Bereich von 15° bis 37°C, insbesonders bevorzugt bei einer Temperatur im Bereich von 20° bis 37°C, insbesondere bei RT, für bis zu 6 Stunden, vorzugsweise bis zu 4 Stunden, besonders bevorzugt 2 Stunden, insbesondere 1 Stunde, insbesondere bevorzugt 1 bis 20 Minuten. Beispielsweise kann die Inkubation 2 bis 120 min bei 4° bis 37°C, bevorzugt für 20 bis 30 min bei 25°C oder 37°C, besonders bevorzugt für 35 min bei 37°C erfolgen. Durch Zusatz von Translationshemmstoffen, wie Rifampicin, kann die Inkubationsdauer verlängert werden, um die Bindungseffizienz zu erhöhen. Nach der spezifischen Erkennung und festen Bindung der Bakterien kann unspezifisch gebundenes Material durch Waschen mit wässrigen Puffer, z.B. mit PBS oder PBS-Tween, vorzugsweise bei neutralem pH-Wert, z.B. bei 50 mM Natriumphosphat, pH 7.0 abgetrennt werden. Den verwendeten Puffern können gegebenenfalls zur Erhöhung der Wasch-Effizienz Detergenzien, z.B. Tween 20, Triton X 100 oder chaotrope Agentien, z.B. Guanidiniumhydrochlorid oder Harnstoff, zugesetzt werden. Dieser Waschschritt kann abhängig vom Probenmaterial mehrmals wiederholt werden.

Nach Abtrennung unspezifisch gebundenen Materials kann die Membran der gebundenen Bakterien durch Zugabe von Detergentien (z.B. Sodiumdodecylsulfat, Octylglukosid), Chemikalien (z.B. Polymyxin B), porenbildenden Polypeptiden (z.B. Nisin, Holin, Mellitin) oder Proteinen (z.B. Lysozym) permeabilisiert, bzw. bei Bedarf (abhängig vom verwendeten Nachweisassay) zerstört werden. Diese Membranpermeabilisierung kann für 5 bis 10 min bei einer Temperatur im Bereich von etwa 10° bis 50°C durchgeführt. Anschließend werden die gebundenen Bakterien nachgewiesen.

Wird die Probe z.B. in einer Durchflußkammer oder an einem Filter mit den immobilisierten Phagen oder Phagenproteinen in Kontakt gebracht, muß sie nach der Bindung der Bakterien an die Phagen oder Phagenproteine nicht zwingend entfernt werden, bevor der Nachweis durchgeführt wird.

Der Nachweis der an Phagen oder Phagenproteine gebundenen Bakterien kann durch die Verwendung eines colorimetrischen Tests durchgeführt werden. Dabei wird z.B. NADH (Bergmeyer & Bernt; Methoden der enzymatischen Analyse, Bergmeyer, U. VCH, Weinheim 1974), β-Galactosidaseaktivität (Apte et al., 1995, Wat. Res. 29, 1803-1806), oder anorganisches Phosphat (Lanzetta et al., 1979, Anal. Biochem., 100, 95-97) nachgewiesen. Diese Tests ermöglichen den Nachweis von mindestens 10⁴ Zellen pro ml, durch Verwendung von Fluoreszenzfarbstoffen kann die Sensititvität auf 10² bis 10³ Zellen pro ml verbessert werden. Die colorimetrischen Tests können generell zum Nachweis der Aktivität intrazellulärer, membranständiger oder periplasmatischer Enzyme, oder von Zellbestandteilen, oder Produkten der Phagenreproduktion, z.B. Phagenproteine oder Phagennukleinsäuren, eingesetzt werden. Die Phagennukleinsäuren können ferner so modifiziert sein, daß in das Phagengenom eine exogene Nukleinsäure, z.B. ein Gen für die Meerrettich-Peroxidase, kloniert ist. Nach Injektion der Phagennukleinsäure in das gebundene Bakterium wird das exogene Gen exprimiert und die Aktivität des Genprodukts kann mit üblichen Verfahren nachgewiesen werden. Ferner kann die exogene Nukleinsäure irgendein bakterienfremdes Polypeptid codieren, das dann nachgewiesen werden kann.

Die colorimetrischen Tests können für alle zu untersuchenden Bakterien gleich, aber auch für bestimmte Bakterien/Phagen-Kombinationen spezifisch sein. Die Messung der enzymatischen Aktivität cytoplasmatischer oder periplasmatischer Enzyme wird, nach einer Membranpermeabilisierung der gebundenen Bakterien durchgeführt. Nachgewiesen wird vorzugsweise die Aktivität ubiquitärer Enzyme, wie z.B. Laktatdehydrogenase oder Proteindisulfidisomerase. Die Enzymauswahl kann an die jeweils getestete Bakteriengattung bzw. gattungsspezifische Fragestellung angepasst werden. Für die colorimetrischen Testsysteme werden je nach benötigter Empfindlichkeit Chemie- oder Biolumineszenz-, Absorptions-, Fluoreszenz- oder Cirkulardichroismusdetektionsverfahren eingesetzt.

Der Nachweis der an Phagen oder an Phagenproteine gebundenen Bakterien kann ferner über den Nachweis von DNA und/oder RNA durchgeführt werden. Dazu können Substanzen verwendet werden, die an DNA und/oder RNA binden. Die Bindung an die DNA und/oder RNA kann direkt auf der Basis einer Membrandiffusion oder alternativ durch eine Membranpermeabilisierung erfolgen. Hier kann auf käufliche Fluoreszenzmarker wie z.B. Ethidiumbromid, Acridinorange, 4',6'-diamidino-2-phenylindole (DAPI) oder SYBR green I zurückgegriffen, und die entsprechenden in der Fachliteratur beschriebenen Detektionsprotokolle verwendet werden.

Der Nachweis der an Phagen oder an Phagenproteine gebundenen Bakterien kann ferner über den Nachweis neuproduzierter DNA und/oder RNA durchgeführt werden. Dazu können membranpermeable, fluoreszenzmarkierte Nukleotide in die neuproduzierte Phagen-DNA und/oder -RNA eingebaut werden.

Ein weiteres Nachweisverfahren ist die Hybridisierung mit fluoreszenzmarkierten, stark konservierten Oligonukleotiden der 16SrRNA (Shine-Dalgarno Sequenz) und die Detektion des Hybridisierungssignales per Fluoreszenz. Bevorzugt ist das Nachweisverfahren unter Verwendung von Phagenproteinen oder Phagen-"Gosts" (Nukleotidfreie, "leere Phagenhüllen"), da damit das Hintergrundsignal durch Phagen-DNA oder -RNA reduziert ist.

Der Nachweis der an Phagen oder an Phagenproteine gebundenen Bakterien kann ferner unter Verwendung von Polypeptiden, z.B. Antikörpern, an die eine Markierung gekoppelt ist, z.B. FITC oder alkalische Phosphatase, gegen Zelloberflächenstrukturen der Bakterien oder von Lektinen gegen Zelloberflächenstrukturen der Bakterien durchgeführt werden, wobei die Signalentwicklung eines z.B. Peroxidase-gekoppelten Antikörpers photometrisch verfolgt wird. Die von den Antikörpern oder den Lektinen erkannten Zelloberflächen der Bakterien können z.B. Lipopolysaccharide oder Membranproteine sein. Die Polypeptide können ferner Phagenproteine sein, die zu den immobilisierten Phagenproteinen identisch oder verschieden sein können. Ferner kann der Nachweis unter Verwendung kompletter Phagen durchgeführt werden, die zu den immobilisierten Phagen identisch oder verschieden sein können.

Das erfindungsgemäße Nachweisverfahren kommt dabei ohne die Verwendung von Zweitantikörpern aus. Im Gegensatz zu dem herkömmlichen ELISA-Verfahren, bei dem die zu untersuchenden Bakterien an ein Trägersystem gekoppelt werden und anschließend die Detektion über Primär- und Sekundärantikörper erfolgt, wird durch Verwendung von an Trägersysteme gekoppelte Phagenpartikel eine Voranreicherung und Selektion durchgeführt. Damit kann die Sensitivität des ELISA-Verfahrens von derzeit 10⁴-10⁶ Keime pro ml (Blasco et al., 1998; J. Appl. Microbiol., 84, 661-666) deutlich gesenkt werden.

Nachgewiesen werden je nach Bedarf z.B. Fluoreszenz, Lumineszenz, Absorption oder Circulardichroismus, Leitfähigkeit oder Kapazitätsänderungen der jeweiligen Proben in den entsprechenden Standardmeßgeräten. Für eine exakte Konzentrationsbestimmung der Bakterien kann eine Eichgerade mit entsprechenden Standardmolekülen durchgeführt werden. Um eine exakte Bestimmung der Bakteriengattung zu erreichen, kann auf eine Reihe von in der Fachliteratur bereits beschriebenen Typisierungsschemata zurückgegriffen werden. Bei Bedarf werden neue Typisierungsschemata, also geeignete Kombinationen verschiedener Phagen, zur exakten Stammbestimmung konstruiert und verwendet.

Die Verwendung des erfindungsgemäßen Verfahrens ermöglicht einen schnellen und sensitiven Nachweis von Bakterien. Durch Kopplung an geeignete, vorstehend beschriebene, Trägerstrukturen wird eine schnelle und kostengünstige Bestimmung einer Vielzahl von Bakterienstämmen, eine sehr genaue Bestimmung der Bakteriengattung und/oder eine Quantifizierung der Bakterien in einem einzigen Test ermöglicht. Unter anderem die genaue Bestimmung der Bakteriengattung ist in der medizinischen Diagnostik auch für die epidemiologische Charakterisierung der Erreger von Bedeutung.

Das erfindungsgemäße Verfahren kann zum schnellen, hochsensitiven und preiswerten Nachweis von Bakterien in allen gewünschten Proben, insbesondere in der Medizin, Lebensmittelindustrie und -analytik, Tierzucht, Trinkwasser- oder Umweltanalytik verwendet werden. Die einfache Durchführbarkeit des Verfahrens ermöglicht sowohl Paketlösungen für die wichtigsten Bakterienkombinationen, als auch an Kundenbedürfnissen angepaßte Systemlösungen und somit einen universellen Einsatz des erfindungsgemäßen Verfahrens. Die vorliegende Erfindung erlaubt ferner eine vollständige Automatisierung des erfindungsgemäßen Verfahrens. Das Verfahren ist ferner für alle Bakterien anwendbar, für die geeignete Phagen vorhanden sind oder in Zukunft isoliert werden, oder für Bakterien, für die durch Selektion entsprechende Typisierungsphagen oder Phagenproteine erzeugt werden können. Das erfindungsgemäße Verfahren bietet außerdem die Möglichkeit zum Einsatz in Bakteriendetektionskits für "jedermann" zum Hausgebrauch.

Ein weiterer Aspekt der Erfindung betrifft somit einen Kit zum Nachweis von Bakterien, umfassend einen Träger mit immobilisierten Phagen oder Phagenproteinen und die für den Nachweis der gebundenen Bakterien notwendigen Lösungen mit den Testreagentien. Die Träger können alle vorstehend beschriebenen Träger sein, an die die Phagen oder Phagenproteine wie vorstehend beschrieben immobilisiert sind. Die Lösungen mit den Testreagentien entsprechen ebenfalls den Substanzen, die für den Nachweis der Bakterien im erfindungsgemäßen Verfahren beschrieben sind. Der Kit kann ferner gegebenenfalls Waschlösungen, sowie für den Aufschluß der Bakterienmembran benötigte Enzyme oder Detergenzien enthalten.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### 1. Isolierung und Reinigung der Phagen

Die Reinigung der E.coli Phagen T4, T7, Qβ und PhiX174 erfolgte nach Anzucht der Phagen auf den entsprechenden, von der DSM angegebenen E.coli-Wirtsbakterien auf der Basis üblicher Verfahren. Um eine vollständige Abtrennung von Bakterien und Bakterienresten zu erreichen, wurde die Phagensuspension niedertourig zentrifugiert (5000 x g, 30 min). Zur Konzentration und Isolierung der Phagen erfolgte eine übliche präparative Ultrazentrifugation und eine Polyethylenglykolfällung. Der Erfolg der Bakterienabtrennung wurde über ein Platierungsexperiment kontrolliert und die Phagen wurden anschliessend gekühlt (4°-8°C) oder gefroren (-20° oder -80°C) gelagert.

### 2. Isolierung und Reinigung der Phagenrezeptorstrukturen

Die Phagenrezeptorstrukturen wurden aus intakten Phagen durch übliche proteinchemische Standardtrennverfahren abgetrennt oder rekombinant produziert und ebenso über proteinchemische Standardtrennverfahren aufgereinigt und wie die kompletten Phagenpartikel gelagert.

### 3. Fixierung der Phagen mittels Adsorption

10⁸-10¹² Phagen/ml wurden in wässrigem Puffer (100mM Tris pH 7,4, 150mM NaCL, 0,03% (w/v) Gelantine oder 50mM Natriumphosphat, pH 7,0) entweder über mehrere Stunden bei 37°C oder über Nacht bei 20° auf Nunc Maxisorb-Platten durch Adsorption direkt immobilisiert. Anschliessend wurden die ungebundenen Phagen durch vierfaches Waschen mit entweder 100mM Tris, pH 7,3 oder 50mM Natriumphosphat pH, 7,5 entfernt.

Anschließend wurde zur Unterdrückung unspezifischer Nebenreaktionen der Bakterien an dem Material des Trägersystems ein Blockierungsschritt durchgeführt. Dazu wurde das phagenbehandelte Trägersystem mit Blockierungslösung PBS (4 mM KH₂PO₄, 16 mM Na₂PO₄, 115 mM NaCl) und dem Zusatz von 0,05-1,0% Tween 20, 1% Rinderserumalbumin über Nacht in einem Temperaturbereich von 4°C oder 2 h bei 37°C inkubiert, die überstehende Flüssigkeit abgenommen und das Trägersystem anschließend wie oben beschrieben getrocknet.

### 4. Fixierung der Phagen mittels kovalenter Bindung

Polystyrolplatten von Nunc (CovaLink™) und Costar (mit NH₂-Gruppen) wurden entsprechend des Herstellerprotokolls mit Cyanurchlorid (48mg in 3 ml Aceton, 45 ml 100mM Natriumphosphat pH 7,0) aktiviert. Dazu wurden 100-200µl Cyanurchlorid innerhalb von zwei Minuten in die Vertiefungen der Platten pipettiert und fünf Minuten bei RT inkubiert. Anschliessend wurde dreimal mit 100mM Natriumphosphat, pH 7,0 gewaschen und für mehr als 30 Minuten bei 50°C getrocknet. Zur Kopplung wurden die Phagen in 100mM Natriümcarbonat, pH 10,0 über Nacht bei RT in den Vertiefungen inkubiert. Durch dreimaliges Waschen mit 50mM Natriumphosphat, pH 7,0 wurden nicht gebundene Phagen entfernt. Die fertigen Platten wurden getrocknet oder bis zur Verwendung mit wässrigem Puffer überschichtet und bei 4°-20°C gelagert.

### 5. Nachweis der gebundenen Bakterien mittels β-Galactosidaseaktivität:

Die Bakterienproben (200µl Probe/Well) wurden für 35 min bei 37°C inkubiert. Anschließend wurden unspezifisch gebundene Bakterien durch dreimaliges Waschen mit entweder je 200 µl 100mM Tris, pH 7,0 oder Phosphatpuffer, pH 7,0 abgetrennt. Für den Farbtest wurden 200µl Waschpuffer mit MgCl₂ und Mercaptoethanol, sowie 66µl ONPG (o-Nitrophenyl-β-D-Galactopyranosid, 4mg/ml) zugesetzt. Der Testansatz wurde bei 37°C inkubiert und der Verlauf der Reaktion bei 405nm über mehrere (2-5) Stunden spektrometrisch verfolgt.

## Patentansprüche

1. Verfahren zum Nachweis von Bakterien, umfassend die folgenden Schritte
a) Kopplung von Bakteriophagen und/oder Bakterienphagenproteinen mittels direkter Bindung an einen Träger oder mittels direkter Bindung der Bakteriophagen und/oder Bakterienphagenproteine an ein an einen Träger immobilisiertes Polypeptid,
b) Inkubieren des mit den Bakteriophagen und/oder Bakterienphagenproteinen gekoppelten Trägers mit einer Probe,
c) gegebenenfalls Entfernen der Probe und der nicht an die Bakteriophagen und/oder Bakterienphagenproteine gebundenen Bakterien der Probe,
d) gegebenenfalls Zugabe von die Bakterienmembran permeabilisierenden oder zerstörenden Substanzen, und
e) Nachweisen der an die Bakteriophagen und/oder Bakterienphagenproteine gebundenen Bakterien der Probe, wobei die gebundenen Bakterien keinem Kultivierungsschritt unterzogen werden.

2. Verfahren nach Anspruch 1, wobei der Nachweis mittels eines colorimetrischen Nachweises von Zellbestandteilen und/oder Produkten der Phagenreproduktion, mittels eines Nachweises von DNA und/oder RNA oder mittels eines Immunnachweises durchgeführt wird.

3. Verfahren nach Anspruch 1 oder 2, wobei die direkt an einen Träger gebundenen Bakteriophagen und/oder Bakterienphagenproteine mittels Adsorption an den Träger oder mittels chemischer Bindung an den Träger gekoppelt sind.

4. Verfahren nach Anspruch 1 oder 2, wobei die an den Träger immobilisierten Polypeptide spezifische Antikörper, Lektine, Rezeptoren oder Anticaline sind.

5. Verfahren nach einem der Ansprüche 1 bis 4, wobei die Bakteriophagen und/oder Bakterienphagenproteine Modifikationen aufweisen.

6. Verfahren nach einem der Ansprüche 1 bis 5, wobei mindestens zwei verschiedene Bakteriophagen und/oder Bakterienphagenproteine verwendet werden, die mindestens zwei verschiedene Bakterienarten und/oder -gattungen erkennen.

7. Verfahren nach einem der Ansprüche 1 bis 6, wobei der Träger eine Mikrotiterplatte, Teststreife, Objektträger, Wafer, Filtermaterial oder eine Durchflußzellkammer ist und aus Polystyrol, Polypropylen, Polycarbonat, PMMA, Celluloseacetat, Nitrozellulose, Glas oder Siliziumwafer besteht.

8. Verwendung des Verfahrens nach einem der Ansprüche 1 bis 7 zur Nachweis von Bakterien in der Medizin, Lebensmittelindustrie und -analytik, Tierzucht, Trinkwasser- oder Umweltanalytik.

9. Kit zur Durchführung des Verfahrens nach einem der Ansprüche 1 bis 7, umfassend einen Träger mit immobilisierten Phagen oder Phagenproteinen und Testreagentien zum Nachweis der gebundenen Bakterien.

10. Kit nach Anspruch 9, ferner umfassend Waschlösungen und/oder die Bakterienmembran permeabilisierende oder zerstörende Substanzen.

## Claims

1. Method for the detection of bacteria, the method comprising the following steps:
a) coupling bacteriophages and/or bacteriophage proteins to a support, either by coupling the bacteriophages and/or bacteriophage proteins via direct binding to a support or via direct binding of the bacteriophages and/or bacteriophage proteins to a polypeptide immobilized to a support,
b) incubating the support coupled with the bacteriophages and/or bacteriophage proteins with a sample,
c) optionally removing the sample and the bacteria in the sample not bound to the bacteriophages and/or bacteriophage proteins,
d) optionally adding substances permeabilizing or destroying the bacterial membrane, and
e) detecting the bacteria in the sample bound to the bacteriophages and/or bacteriophage proteins, wherein the bound bacteria are not subjected to a cultivation step.

2. Method according to claim 1, wherein detection is performed by means of a colorimetric detection of cell components and/or products of the phage reproduction, by means of a detection of DNA and/or RNA or by means of an immunoassay.

3. Method according to claim 1 or 2, wherein the bacteriophages and/or bacteriophage proteins directly bound to a support are coupled to the support by means of adsorption or by means of chemical binding.

4. Method according to claim 1 or 2, wherein the polypeptides immobilized to the support are specific antibodies, lectins, receptors, or anticalins.

5. Method according to any of claims 1 to 4, wherein the bacteriophages and/or bacteriophage proteins exhibit modifications.

6. Method according to any of claims 1 to 5, wherein at least two distinct bacteriophages and/or bacteriophage proteins recognizing at least two distinct species and/or genera of bacteria are used.

7. Method according to any of claims 1 to 6, wherein the support is a microtiter plate, a test stripe, a slide, a wafer, a filter material, or a flow-through cell chamber consisting of polystyrene, polypropylene, polycarbonate, PMMA, cellulose acetate, nitrocellulose, glass, or silicium wafer.

8. Use of the method according to any of claims 1 to 7 to detect bacteria in the areas of medicine, food industry and analytics, livestock breeding, fresh water or environmental analytics.

9. Kit to perform the method according to any of claims 1 to 7, the kit comprising a support with immobilized phages or phage proteins and assay reagents to detect the bound bacteria.

10. Kit according to claim 9, further comprising washing solutions and/or substances permeabilizing or destroying the bacterial membrane.

## Revendications

1. Procédé permettant la mise en évidence de bactéries, comprenant les étapes suivantes :
a) Couplage de bactériophages et/ou de protéines de bactériophages par liaison directe sur un support ou par liaison directe des bactériophages et/ou protéines de bactériophages sur un polypeptide immobilisé sur un support,
b) Incubation du support, couplé aux bactériophages et/ou protéines de bactériophages, avec un échantillon,
c) Elimination, le cas échéant, de l'échantillon et des bactéries de l'échantillon non liées aux bactériophages et/ou aux protéines de bactériophages,
d) Le cas échéant, addition de substances rendant perméable ou détruisant la membrane des bactéries, et
e) Mise en évidence des bactéries de l'échantillon liées aux bactériophages et/ou aux protéines de bactériophages, les bactéries liées n'étant soumises à aucune étape de culture.

2. Procédé selon la revendication 1, dans lequel la mise en évidence est réalisée par une mise en évidence colorimétrique de constituants cellulaires et/ou de produits de la reproduction de phages, par une mise en évidence de l'ADN et/ou de l'ARN ou par un test immunologique.

3. Procédé selon la revendication 1 ou 2, dans lequel les bactériophages et/ou protéines de bactériophages directement liés à un support sont couplés au support par adsorption ou par liaison chimique au support.

4. Procédé selon la revendication 1 ou 2, dans lequel les polypeptides immobilisés sur le support sont des anticorps spécifiques, des lectines, des récepteurs ou des anticalines.

5. Procédé selon l'une quelconque des revendications 1 à 4, dans lequel les bactériophages et/ou les protéines de bactériophages présentent des modifications.

6. Procédé selon l'une quelconque des revendications 1 à 5, dans lequel au moins deux bactériophages et/ou protéines de bactériophages différents sont utilisés qui reconnaissent au moins deux types et/ou espèces différents de bactéries.

7. Procédé selon l'une quelconque des revendications 1 à 6, dans lequel le support est une plaque de microtitration, une bande de test, un porte-objet, une lame de silicium, un matériau filtrant ou une chambre bactériologique à écoulement, et est constitué de polystyrène, de polypropylène, de polycarbonate, de PMMA, d'acétate de cellulose, de nitrocellulose, de verre ou de silicium.

8. Utilisation du procédé selon l'une quelconque des revendications 1 à 7 pour la mise en évidence de bactéries en médecine, dans l'industrie alimentaire et pour l'analyse des aliments, l'élevage d'animaux, l'analyse de l'eau potable et l'analyse environnementale.

9. Kit pour la réalisation du procédé selon l'une quelconque des revendications 1 à 7, comportant un support avec des phages ou des protéines de phages immobilisés et des réactifs de test pour la mise en évidence des bactéries liées.

10. Kit selon la revendication 9, comprenant par ailleurs des solutions de lavage et/ou des substances rendant perméable ou détruisant la membrane des bactéries.
